# EUROPEAN PATENT APPLICATION

(11) **EP 1 913 949 A1**
(43) Date of publication of application: **23.04.2008**
(21) Application number: 06122067.9
(22) Date of filing: 11.10.2006
(51) Int. Cl.: A61K 33/36, A61P 9/04

(54) **Modulation of substrate preference by selective translocation of the major cardiac glucose transporter Glut4**

(71) Applicant: Universiteit Maastricht, 6229 ER Maastricht (NL)
(72) Inventor: Glatz, Jan, 6213 GJ, Maastricht (NL); Luiken, Joost, 6227 BR, Maastricht (NL)
(74) Representative: Habets, Winand

(57) **Abstract**

This invention is in the field of molecular medicine and provides new leads for anti-diabetic therapies. More in particular it relates to a method for specifically increasing glucose uptake in a muscle cell wherein a stimulus is provided to said muscle cell upon which stimulus the major cardiac glucose transporter GLUT4 is translocated from the GLUT4 storage compartment directly to the sarcolemma, thereby by-passing the insulin-responsive recycling endosomes, wherein said stimulus does not alter the uptake of long-chain fatty acids in said muscle cell.

## Description

### Field of the Invention

This invention is in the field of molecular medicine and provides new leads for anti-diabetic therapies. More in particular it relates to a method for specifically increasing glucose uptake in a muscle cell wherein a stimulus is provided to said muscle cell upon which stimulus the major cardiac glucose transporter GLUT4 is translocated from the GLUT4 storage compartment directly to the sarcolemma, thereby by-passing the insulin-responsive recycling endosomes, wherein said stimulus does not alter the uptake of long-chain fatty acids in said muscle cell.

### Background of the invention

Glucose and fatty acids are the main fuels for energy production in cardiac and skeletal muscle. The uptake of these substrates by muscle cells occurs via specific transporter proteins present in the muscle membrane, i.e., the glucose transporter GLUT4 and the fatty acid transporter CD36. These transporters are stored inside the cell and upon a proper stimulus - such as muscle contraction - translocate to the cell membrane to increase glucose and fatty acid uptake. Recent studies indicate that GLUT4 is present in a so-called storage compartment from where it first migrates to a so-called endosomal compartment before being recruted to the cell membrane. CD36 is also present in this endosomal compartment and from there directly translocates to the cell membrane.

In diabetes patients glucose uptake by muscle tissue and other tissues is impaired resulting in an elevated plasma glucose concentration. By virtue of its large mass (35% of total body mass) skeletal muscle is a main regulator of plasma glucose. The current technology to treat diabetes consists of (i) the use of general antidiabetic agents such as biguanides (e.g. metformin) and glucosidase inhibitors which slow down intestinal glucose uptake and increase muscle glucose uptake, and (ii) thiazolidinediones (e.g. Rosiglitazone) which also increase muscle glucose uptake.

These antidiabetic therapies have the disadvantage that most of them show complications while all are only modestly effective. Thus, biguanides increase muscle glucose uptake by stimulating the translocation of GLUT4 to the cell membrane, which is mediated by activation of AMP-dependent kinase. However, activation of AMP-kinase also translocates CD36 to the cell membrane, resulting in increased fatty acid uptake. The excess fatty acids are stored in the cell and gradually impair cellular function, which is referred to as lipotoxicity. Cardiac malfunction is common in diabetes patients ('diabetic cardiomyopathy') and may explain why the majority of diabetes patients die from cardiovascular disease.

Thiazolidinediones are only modestly effective because the (nuclear) target for these agents is present in muscle in minute amounts. A complication is that these agents affect adipose tissue to cause obesity. In addition, these agents increase both muscle glucose and fatty acid uptake, leading to excessive lipid storage as explained above.

Taken together, there is an urgent need for agents that would selectively increase glucose uptake without affecting muscle fatty acid uptake.

### Summary of the invention

Surprisingly, it has now been found that it is possible to uncouple glucose and fatty acid uptake in muscle tissue. In particular we found that it is possible to selectively translocate the major cardiac glucose transporter GLUT4 to the cell membrane, without affecting subcellular localization of CD36. Even more in particular, we found that the protein thiol-modifying agent arsenite selectively recruits GLUT4 to the muscle cell membrane without affecting CD36. As a result, glucose uptake is stimulated while fatty acid uptake remains unaltered.

We were able to show that GLUT4 is recruited directly from the GLUT4 storage compartment inside the muscle cell, circumventing the common route of GLUT4 via the endosomal compartment. Because this GLUT4 storage compartment does not contain CD36, this explains the selective increase in glucose uptake without affecting fatty acid uptake.

Hence, the invention relates to a method for specifically increasing glucose uptake in a muscle cell wherein a stimulus is provided to said muscle cell upon which stimulus the major cardiac glucose transporter GLUT4 is translocated from the GLUT4 storage compartment directly to the sarcolemma, thereby by-passing the insulin-responsive recycling endosomes, wherein said stimulus does not alter the uptake of long-chain fatty acids in said muscle cell.

This invention also encompasses the stimulation of an arsenite sensitive protein within or closely associated with the GLUT4 storage compartment that upon thiol-modification initiates the translocation of GLUT4 from this compartment directly to the cell membrane. This intracellular target of arsenite (ITA) is therewith a valuable target for novel anti-diabetic therapy.

The advantage of the invention is that increased muscular glucose uptake lowers blood glucose concentration thereby reducing the production of so-called advanced glycation end products which are a major complication of diabetes.

Abbreviations used herein are: ACC, acetyl-coenzyme A carboxylase; AMPK, AMP-activated protein kinase: BSA, bovine serum albumin; LCFA, long-chain fatty acids; CD36, human homolog of rat fatty acid translocase; ERK, extracellular signal-regulated kinase; FABPpm, plasma membrane fatty acid-binding protein; JNK, c-JUN NH₂-terminal protein kinase; LDM, low-density microsomal; MAPK, mitogen-activated protein kinase; PI3K, phosphatidylinositol-3 kinase; PKB, protein kinase B; PM, plasma membrane; S6K, S6 kinase.

### Figure Legends

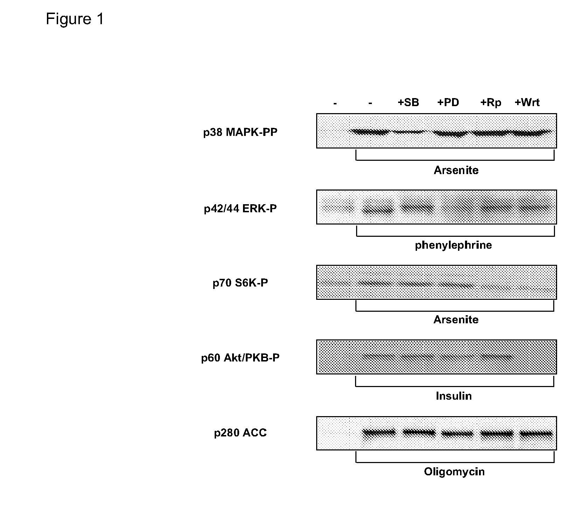

Figure 1. Verification of effectiveness and specificity of the applied signaling inhibitors on inhibition of protein kinase phosphorylation in cardiac myocytes. Cell suspensions were pre-incubated for 20 min with signaling inhibitors (10 µM SB202190, 10 µM PD98059, 20 nM rapamycin or 200 NM wortmannin), and subsequently incubated for 20 min with metabolic stimuli (100 µM arsenite, 50 µM phenylephrine, 100 nM insulin or 5 µM oligomycin). To stop the incubations, sample buffer was added, and samples were used for gel electrophoresis and Western blotting for the detection of diphospho-p38 MAPK, diphospho-p42/44 ERK, phospho-p70 S6K, phospho-p60 Akt/PKB or phospho-p280 ACC as detailed in Materials and Methods. A representative Western blot is shown out of 3 experiments carried out with different cardiomyocyte preparations.

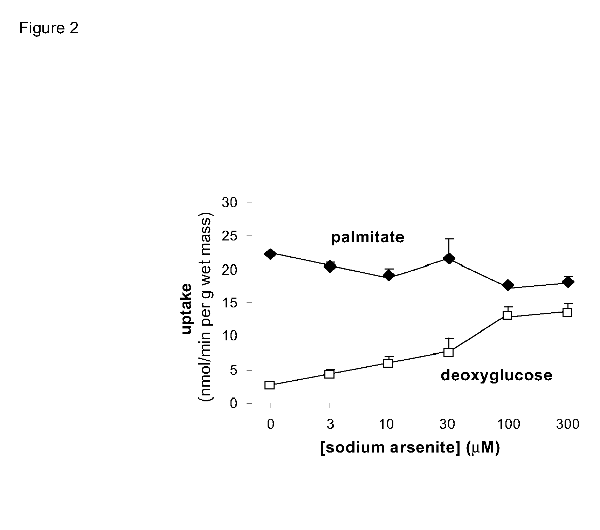

Figure 2. Dose dependent effects of arsenite on glucose and LCFA uptake into cardiac myocytes. Cell suspensions were incubated for 20 min with various concentrations of arsenite, and subsequently used for measurement of uptake of palmitate and 2-deoxy-D-glucose. Data are means ± S.E.M of 3 experiments carried out with different cardiomyocyte preparations. □: deoxyglucose uptake; ◆: palmitate uptake.

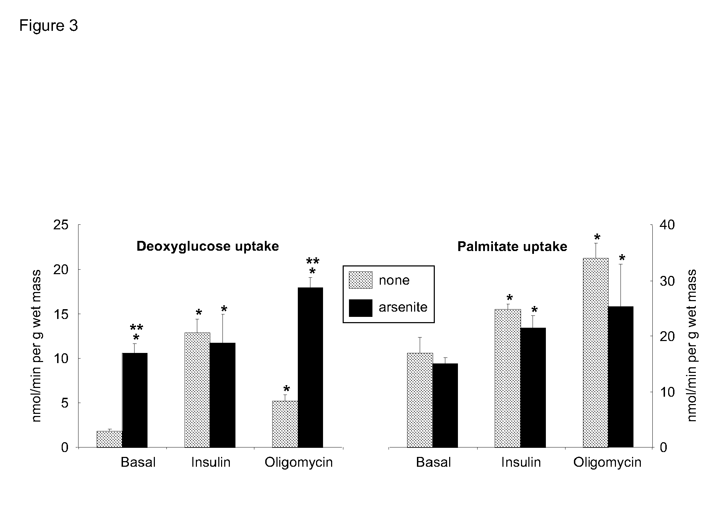

Figure 3. Effects of arsenite on glucose and LCFA uptake into cardiac myocytes: comparison with insulin and oligomycin. Cell suspensions were incubated in the absence of additions (Basal) or presence of 100 µM arsenite, 100 nM insulin, 5 µM oligomycin (Oli), or combinations of these stimuli for 20 min prior to execution of 2-deoxy-D-glucose uptake studies (3 min; left panel) or palmitate uptake studies (3 min; right panel). Grey bars: incubations in absence of arsenite; black bars: incubations in the presence of arsenite. Data are means ± S.E.M. of 4-6 experiments carried out with different cardiomyocyte preparations. *Significantly different from myocytes incubated without any additions (basal/none) (*P* < 0.05). **Significantly different from corresponding myocytes incubated in the absence of arsenite (none) (*P* < 0.05).

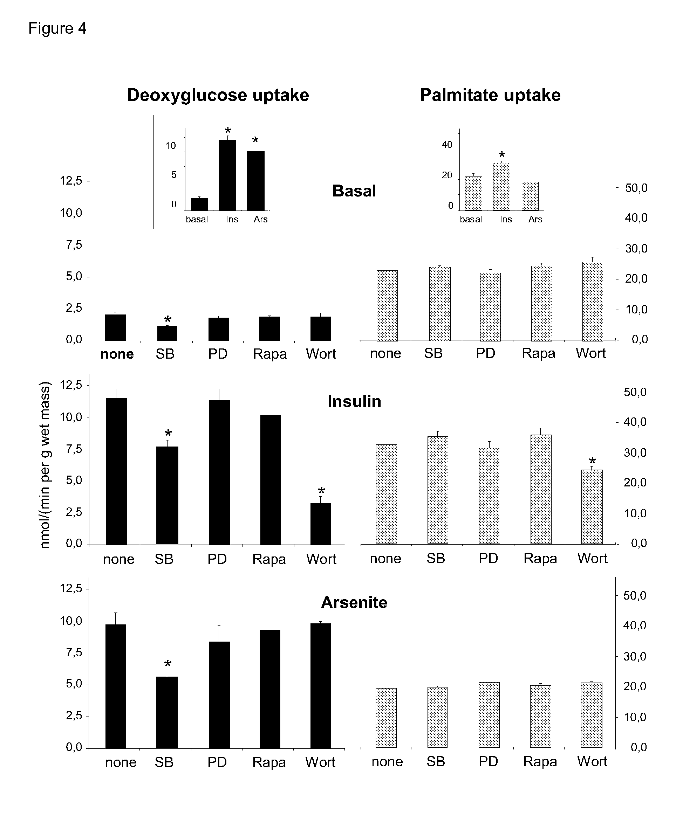

Figure 4. Effects of signaling inhibitors on arsenite- and insulin-induced glucose and LCFA uptake. Cell suspensions were pre-incubated for 20 min with signaling inhibitors (10 µM SB202190, 10 µM PD98059, 20 nM rapamycin or 200 NM wortmannin, i.e., SB, PD, Rapa or Wort, respectively), and subsequently incubated for 20 min without (referred to as basal; the two upper panels) or with 100 nM insulin (the two middle panels) or 100 µM arsenite (the two lower panels). Immediately thereafter, cells were subjected to 2-deoxy-D-glucose uptake studies (3 min; the three left panels) or palmitate uptake studies (3 min; the three right panels). The two inserts represent the effects of arsenite or insulin on glucose uptake (right insert) and LCFA uptake (left insert) in the absence of inhibitors. The data in the inserts are from the same experiments as indicated under 'none'. Data are means ± S.E.M. of 3-4 experiments carried out with different cardiomyocyte preparations. *Significantly different from myocytes without additions (none) (*P* < 0.05).

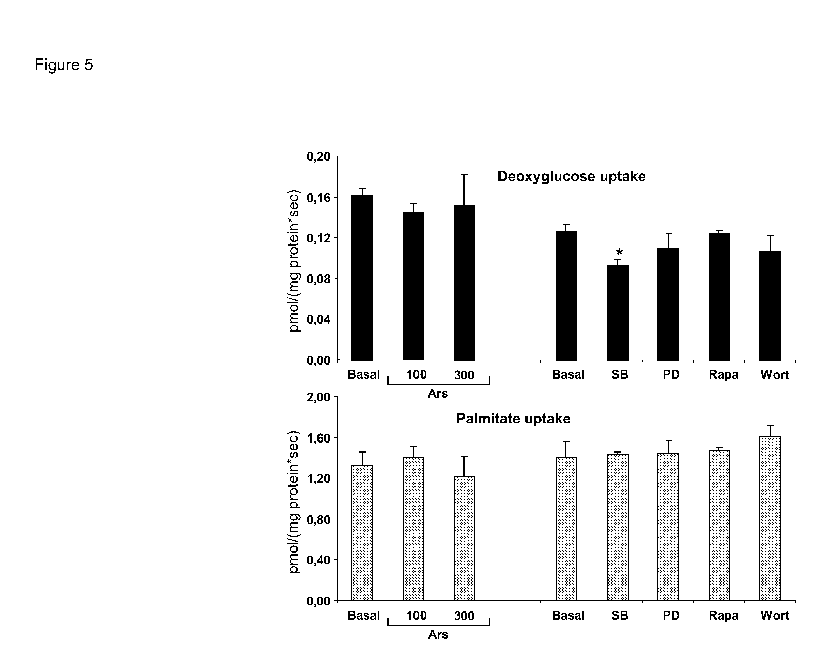

Figure 5. Effects of arsenite on intrinsic activity of substrate transporters in heart-derived giant vesicles. Giant vesicle suspensions were incubated with arsenite or with signaling inhibitors (10 µM SB202190, 10 µM PD98059, 20 nM rapamycin or 200 NM wortmannin), prior to execution of glucose uptake studies (2 min; upper panel) or palmitate uptake studies (15 sec; lower panel). Data are means ± S.E.M. of 3-4 experiments carried out with different cardiomyocyte preparations. *Significantly different from myocytes without additions (Basal) (*P* < 0.05).

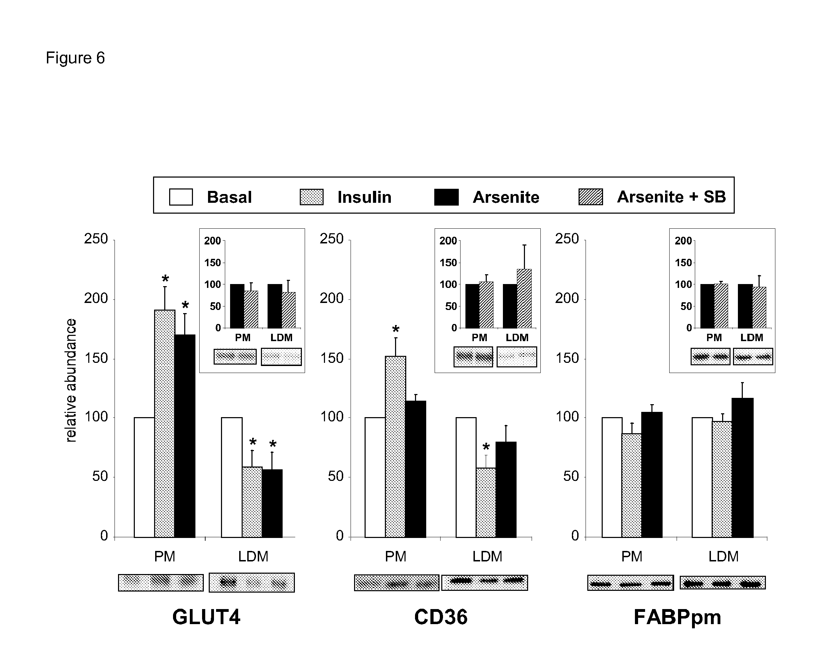

Figure 6. Effects of arsenite on translocation of GLUT4, CD36 and FABPpm: comparison with effects of insulin. Cell suspensions were incubated for 20 min in the absence (Basal) and presence of 100 nM insulin or 100 µM arsenite, after which NaN₃ was added to stop ATP-demanding processes. Immediately hereafter, cells were frozen in liquid nitrogen, and upon thawing, subjected to subcellular fractionation. The collected fractions were analyzed on the relative contents of GLUT4 (45 kDa), CD36 (88 kDa), and FABPpm (43 kDa). Transporter content was expressed as multiple of control (Basal; white bars) in the corresponding fraction. Data are means ± S.E.M. of 6 experiments carried out with different cardiomyocyte preparations. A second set of experiments was performed to investigate the influence of SB202190 on the arsenite-induced changes in transporter recycling. 10 µM SB202190 was added to cell suspensions 20 min prior to addition of arsenite. Transporter content was expressed as multiple of values obtained in cell incubations with arsenite (black bars; see inserts). Data are means ± S.E.M. of 4 experiments carried out with different cardiomyocyte preparations. PM, plasma membrane fraction; LDM, low density microsomal fraction; SB, SB202190. Representative Western blots are shown. *Significantly different from myocytes in the absence of additions (Basal) (*P* < 0.05).

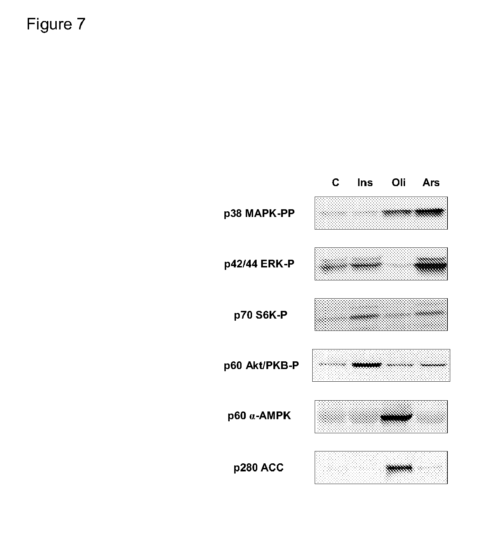

Figure 7. Effects of arsenite on activation of signaling enzymes. comparison with insulin and oligomycin. Cell suspensions were incubated for 20 min with 100 nM insulin, 5 µM oligomycin or 100 µM arsenite. At the stop of the incubations, sample buffer was added, and samples were used for gel electrophoresis and Western blotting for the detection of diphospho-p38 MAPK, diphospho-p42/44 ERK, phospho-p70 S6K, phospho-p60 Akt/PKB or phospho-p280 ACC as detailed in Materials and Methods. A representative Western blot is shown out of 3-6 experiments carried out with different cardiomyocyte preparations.

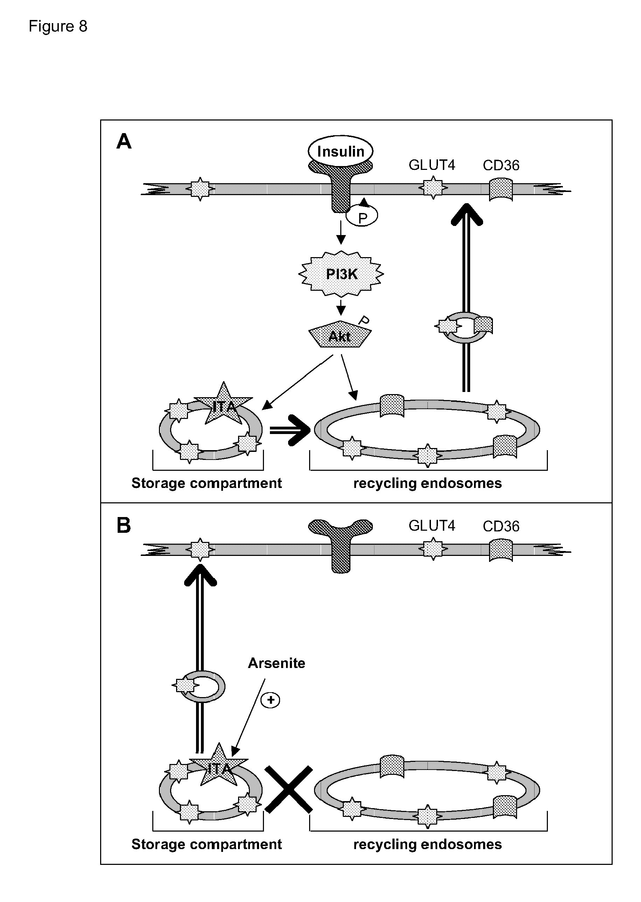

Figure 8. Putative scheme of arsenite's action on transporter recycling, comparison with those of insulin and contractions. Under basal conditions, the majority of GLUT4 and ~50% of CD36 is present in intracellular stores with the remaining portion of both transporters at the sarcolemma. Intracellularly, GLUT4 is present in both the storage compartment and the recycling endosomes, whereas the localization of CD36 is restricted to the recycling endosomes. Panel A: Insulin, through activation of PI3K and subsequently of Akt/PKB mobilizes GLUT4 from the storage compartment, and also both GLUT4 and CD36 from insulin-responsive recycling endosomes. GLUT4 mobilized from the storage compartment and on its way to the sarcolemma travels through the recycling endosomes, from where its shares its transport vesicle together with CD36. Panel B: Arsenite does not affect the dynamics of the recycling endosomes, but selectively recruits GLUT4 from the storage compartment through sulfhydryl-modification of an unknown protein (intracellular target of arsenite: ITA) within this compartment. Vesicles excised by "thiol-activation" of ITA shortcut the endosomal route but migrate directly to the sarcolemma.

### Detailed description of the invention

The protein thiol-modifying agent arsenite, a potent activator of stress signaling, was used to examine the involvement of MAPK's in the regulation of cardiac substrate uptake. Arsenite strongly induced p38 MAPK phosphorylation in isolated rat cardiac myocytes, but also moderately enhanced phosphorylation of p42/44 ERK and p70 S6K. At the level of cardiomyocytic substrate utilization, arsenite enhanced glucose uptake dose-dependently up to 5.1-fold, but failed to stimulate long-chain fatty acid (LCFA) uptake. At the substrate transporter level, arsenite stimulated the translocation of GLUT4 to the sarcolemma but failed to recruit CD36 or FABPpm. Because arsenite did not influence the intrinsic activity of glucose transporters, GLUT4 translocation is entirely responsible for the selective increase in glucose uptake by arsenite. Moreover, the non-additivity of arsenite-induced glucose uptake and insulin-induced glucose uptake indicates that arsenite recruits GLUT4 from insulin-responsive intracellular stores. Inhibitor studies with SB203580/SB202190, PD98059 and rapamycin indicate that activation of p38 MAPK, p42/44 ERK and p70 S6K, respectively, are not involved in arsenite-induced glucose uptake. In addition, all these kinases do not play a role in regulation of cardiac glucose and LCFA uptake by insulin. Hence, arsenite's selective stimulation of glucose uptake appears unrelated to its signaling actions, suggesting that arsenite acts via thiol-modification of a putative intracellular protein target of arsenite within insulin-responsive GLUT4-containing stores. Because of arsenite's selective stimulation of cardiac glucose uptake, identification of this putative target of arsenite within the GLUT4-storage compartment may indicate whether it is a target for future strategies in prevention of diabetic cardiomyopathy.

In the healthy heart, long-chain fatty acids (LCFA) and glucose contribute 70% and 20%, respectively, to cardiac energy production (1). During development of cardiac hypertrophy due to pressure overload, there is a gradual decrease in FA utilization which is partly compensated for by an increase in glucose utilization (2). Conversely, during the development of diabetic cardiomyopathy, a reciprocal shift towards increased utilization of LCFA has been observed (3). These shifts in substrate preference, towards either glucose utilization or towards fatty acid utilization, are accompanied by impaired cardiac functioning (2, 4). Hence, optimal cardiac performance appears dependent on maintaining the ratio between glucose utilization and LCFA utilization within a certain optimal range. Furthermore, modulation of cardiac substrate preference aimed at normalizing the balance between utilization of glucose and LCFA might counteract the development and/or regress cardiac failure.

GLUT4 is the major cardiac glucose transporter whereas the bulk of LCFA uptake is mediated by the concerted action of two structurally unrelated proteins, i.e., an 88 kDa heavily glycosylated fatty acid translocase, referred to as CD36 (5) and a 43 kDa plasma membrane fatty acid binding protein (FABPpm) (6). Both proteins are involved in LCFA uptake and are presumed to make up a functional transport system in which FABPpm is suggested to operate as a receptor for LCFA, and CD36 to operate as a flippase (7). In cardiac myocytes, uptake rates of glucose and long-chain fatty acids (LCFA) are similarly regulated, by inducing the translocation of GLUT4 and CD36, respectively, from intracellular stores to the sarcolemma. Insulin and contractions are two major physiological stimuli able to induce translocation of both transporters, and hence, enhance the uptake of both substrates (8). The similarity between regulation of glucose uptake and of LCFA uptake also extends to the signaling pathways involved. Insulin-induced translocation of both GLUT4 and CD36 has been found to be dependent on activation of phosphatidylinositol-3 kinase (PI3K), whereas AMPK activation is necessary for contraction-induced translocation of both transporters (8).

Whether GLUT4 and CD36 reside in the same intracellular storage compartment has not yet been determined. However, it appears to be possible to uncouple the simultaneous translocation of both transporters from each other. Notably, the cardiotonic agent dipyridamole induced the translocation of CD36 to the sarcolemma of cardiac myocytes without affecting subcellular localization of GLUT4, resulting in a selective increase in LCFA uptake (9). Dipyridamole exerts this effect via a yet unidentified intracellular target, which is situated in the contraction signaling cascade downstream of AMPK. This finding may suggest that CD36 and GLUT4 are stored in separate intracellular pools, or that, alternatively, both transporters reside within the same compartment but are recruited via distinct sorting mechanisms. In theory, dipyridamole, because of its ability to shift cardiac substrate utilization towards LCFA, could be used to improve cardiac functioning during high pressure-induced cardiac hypertrophy. Conversely, a strategy to induce the translocation of GLUT4 without moving CD36 should selectively stimulate glucose uptake, and thereby have antidiabetic potential.

Arsenite is recognized to stimulate glucose uptake in a number of mammalian cell types (10, 11). However, its effect on LCFA uptake is completely unknown. There is evidence, in adipocytes (11) and skeletal muscle cells (10), that the increase in glucose uptake by arsenite involves a stimulation of the intrinsic activity of glucose transporters, eventually in combination with a translocation of GLUT4 to the plasma membrane.

Besides activating glucose uptake, arsenite is also a potent activator of certain signaling kinases. It is known to activate P38 MAPK (12) and P70 S6K (13) in cardiac myocytes. The mechanism of action likely includes modification of vicinal sulfhydryl moieties in a number of limited arsenite-sensitive target proteins (14). The stimulatory effect of arsenite on P38 and on JNK, another related stress kinase, is probably due to modulating the activity of an unknown target protein (15), while arsenite's effect on activation of P70 S6K is incompletely resolved (13). It has been speculated that P38 MAPK could be implicated in the stimulation of glucose uptake into adipocytes based on the partial inhibition of arsenite-induced glucose uptake by the specific MAPK inhibitor SB203580 (11, 16).

In the heart, the roles of p38 MAPK and related ERKs in substrate uptake have scarcely been explored. Interestingly, recent observations pinpoint p38 downstream of AMPK upon low flow- (17) or chemically induced ischemia (18) in the heart. Also p42/44 ERK has been positioned downstream of AMPK in contracting muscle preparations (19). Combined with the above mentioned findings in adipocytes, a complex picture emerges in the way MAPKs are involved in cellular substrate utilization. Nevertheless, elucidation of the role of MAPKs in substrate utilization by the heart is important, when considered that both p38 and ERK are involved in the regulation of cardiac contractility (20, 21).

In the present study we used the ability of arsenite to stimulate MAPKs in order to assess the roles of these kinases in the regulation of substrate uptake by cardiac myocytes. In addition, we also examined the potential induction of MAPKs by insulin and by the contraction-mimetic agent oligomycin. In contrast to many studies that have focussed solely on the link between signaling and cardiac glucose uptake (e.g., 10, 11, 16), we investigated the uptake of both glucose and of LCFA. Notably, the comparison of the effects of arsenite on the regulation of GLUT4 translocation compared to CD36 recycling can shed new light on the role of MAPKs in cardiac substrate utilization. In order to couple signaling to glucose and LCFA uptake we tested the ability of specific MAPK inhibitors to influence the uptake of both substrates into cardiac myocytes in the absence and presence of arsenite, insulin and oligomycin. Collectively, the findings indicate that arsenite specifically stimulates glucose uptake by recruiting GLUT4 from the insulin responsive non-endosomal GLUT4-specific storage compartment. In addition, neither MAPKs nor S6Ks play a role in regulation of uptake of glucose and of LCFA.

Hence, the invention relates to a method for specifically increasing glucose uptake in a muscle cell wherein a stimulus is provided to said muscle cell upon which stimulus the major cardiac glucose transporter GLUT4 is translocated from the GLUT4 storage compartment directly to the sarcolemma, thereby by-passing the insulin-responsive recycling endosomes, wherein said stimulus does not alter the uptake of long-chain fatty acids in said muscle cell.

The main purpose of this study was to assess the role of MAPKs in the regulation of cardiac glucose and LCFA uptake using arsenite as a tool to stimulate cardiac signaling. The following novel findings emerged from arsenite-treated cardiac myocytes in relation to signaling events and substrate uptake: (i) Arsenite stimulates glucose uptake into cardiac myocytes without affecting LCFA uptake. As a mechanism for this selective stimulation of glucose uptake, the present findings indicate that arsenite appears to recruit GLUT4 directly from the non-endosomal GLUT4-specific storage compartment. (ii) The insulin-mimetic action of arsenite on glucose uptake appeared independent of its activation of p38 MAPK, because insulin did not activate p38. (iii) arsenite-induced signaling is likely not involved in arsenite's selective stimulation of glucose uptake. Besides these main findings, other novel observations were made in the course of this study. (iv) The specific MAPK inhibitors SB203580 and SB202190 cannot be used to study the relation between MAPK signaling and glucose uptake due to direct interaction of these two SBs with cell surface cardiac GLUTs. (v) The signaling enzymes p38 MAPK, p42/44 ERK and p70 S6K are neither involved in the short-term regulation of glucose uptake/GLUT4 translocation, nor in LCFA uptake/CD36 translocation by insulin.

Arsenite appears the first compound characterized to be able to induce GLUT4 translocation without altering the subcellular localization of CD36. Previously, we established in heart and skeletal muscle that many stimuli (e.g., insulin, electrical stimulation, oligomycin and AICAR) are able to induce GLUT4 translocation, as well as increase CD36 translocation, leading to a general increase in substrate uptake in these tissues (8). While these observations suggest that GLUT4 translocation and CD36 translocation appear to occur concomitantly, further studies, however, demonstrated that the coupling beween GLUT4 translocation and CD36 translocation appears to be less coordinated. Recently, selective translocation of CD36 was observed in the presence of the cardiotonic agent dipyridamole without alteration in the subcellular distribution of GLUT4, resulting in a selective increase in cardiac LCFA uptake (9). In the present study, selective GLUT4 translocation was observed in the presence of arsenite without alteration in the subcellular distribution of CD36. As a result, it is possible to modulate cardiac substrate preference via selective recruitment of substrate transporters.

The invention therefore also relates to a method as described above wherein a stimuls is provided that targets the intracellular target of arsenite in or in close association with the GLUT4 storage compartment.

Arsenite has been previously shown to stimulate glucose uptake and GLUT4 translocation in adipocytes and skeletal muscle cell lines (10, 11). However, its selective action on glucose uptake but not on LCFA uptake, as observed in the present study, is a novel observation. Moreover, since arsenite did not alter the intrinsic activity of glucose transporters (Figure 5), GLUT4 translocation (Figure 6) must be fully responsible for arsenite's selective stimulation of glucose uptake.

The arsenite-stimulated glucose uptake was completely additive to oligomycin-stimulated glucose uptake. This indicated that arsenite does not recruit GLUT4 from the intracellular contraction-responsive compartment(s). Since AMPK activation by electrical stimulation or by oligomycin has also been found to induce CD36 translocation and LCFA uptake (26), activation of the contraction/AMPK-sensitive signaling pathway does not confer specific substrate selection for either glucose or LCFAs. As further support for this, we observed that, in contrast to the marked oligomycin-induced phosphorylation of AMPK and ACC, indeed, arsenite did not increase the phosphorylation of these two protein kinases, as was observed with oligomycin.

As opposed to arsenite's fully retained potential to stimulate glucose uptake in oligomycin-treated cardiac myocytes, arsenite failed to stimulate glucose uptake further in insulin-stimulated cardiac myocytes. This shows that arsenite mobilizes GLUT4 from the insulin-responsive stores, and not from intracellular contraction-responsive compartments.

Because the kinetic evidence (i.e., non-additivity of insulin-stimulated glucose uptake with arsenite-stimulated glucose uptake) indicates that arsenite interacts with the insulin-stimulated GLUT4 translocation process, we investigated the ability of arsenite to activate signaling enzymes that have been reported to be activated by insulin.
*• Akt*/*PKB:* The phosphorylation of Akt/PKB is a classical insulin response, and is firmly established to be involved in GLUT4 translocation (31). Our evidence also implicates the PI3K-Akt/PKB axis to be responsible for CD36 translocation (8). Thus, this PI3K-Akt/PKB signaling pathway, just as the AMPK pathway, is not solely involved in the preferential uptake of glucose. Moreover, arsenite did not stimulate Akt/PKB phosphorylation, and inhibition of the PI3K-Akt/PKB axis by wortmannin (see Figure 1) did not abrogate the arsenite-stimulated glucose uptake (Figure 4). Altogether, these observations firmly exclude Akt/PKB to be involved in arsenite-stimulated GLUT4 translocation.
*• P38 MAPK:* Phosphorylation of 38 MAPK is the classical signaling response to arsenite exposure, and has also been implicated in insulin-stimulated glucose uptake. Notably, Klip and coworkers showed that insulin stimulated both Akt/PKB and p38 MAPK activation in adipocytes (16). The activation of both kinases was shown to occur by independent pathways, and they contributed independently to the large increase in insulin-stimulated glucose uptake in adipocytes (16, 27). Specifically, Akt/PKB activation is causally related to GLUT4 translocation, and p38 MAPK activation is responsible for an increase in the intrinsic activity of GLUT4. However, whether this activation of GLUT4 involves a phosphorylation or another kind of protein modification has not yet been established. A large pillar in this hypothesis of dual activation of glucose transport was the ability of the widely used p38 MAPK inhibitor SB203580 to partly block insulin-stimulated glucose uptake. Because this compound does not affect insulin-induced GLUT4 translocation, it was, by default, expected to block GLUT4 activation by insulin (16, 27). However, the present study shows that insulin does not stimulate p38 MAPK phosphorylation in cardiac myocytes. This inability of insulin to activate cardiac p38 MAPK, cannot be due to defective MAPK signaling because other stimuli such as arsenite and oligomycin potently stimulate phosphorylation of this serine/threonine kinase (Figure 7). The discrepancy between insulin-induced p38 MAPK activation in adipocytes, as observed by Klip and coworkers (16) and also by others (32), on one hand, and the lack of p38 MAPK phosphorylation upon insulin exposure to cardiac myocytes, as observed in the present study, on the other hand, might be attributed to a tissue-specific action of insulin, although the ability of insulin to stimulate p38 MAPK in adipocytes has now also been disputed recently by others (33). With respect to cardiac myocytes, the lack of stimulation of p38 MAPK by insulin, as observed by us, is in agreement with results in earlier studies in primary cardiac myocyte cultures (34).

The inability of insulin to stimulate p38 MAPK in cardiac myocytes does not rule out the involvement of p38 MAPK in the insulin-mimetic action of arsenite on glucose uptake. In this scenario, arsenite-induced p38 MAPK activation could be part of a signaling pathway parallel to the PI3K/Akt/PKB axis, resulting in the activation of a downstream component that is also activated by Akt/PKB. Nonetheless, we were unable to link arsenite-induced p38 MAPK phosphorylation to arsenite-induced GLUT4 translocation. More specifically, the use of SB202190 and SB203580 indicates that p38 MAPK is not involved in arsenite-stimulated glucose uptake, notwithstanding the observation that both compounds partially inhibited arsenite-stimulated glucose uptake. Notably, both SB's partially inhibited glucose uptake in cardiac myocytes during arsenite stimulation, during insulin stimulation as well as in unstimulated quiescent cardiac myocytes. However, in quiescent cardiac myocytes and during insulin stimulation p38 MAPK is not activated (Figure 7). Moreover, the inhibitory effect of both SB's on glucose uptake is completely retained in giant vesicles (Figure 5), adding powerful evidence that these compounds directly interfere with the transport function of GLUT's that are located at the plasma membrane. Hence, because the magnitude of inhibition exerted by both SB's on glucose uptake is very similar between giant vesicles and arsenite-stimulated cardiac myocytes, the direct interaction of SB's with GLUT4 fully accounts for their inhibitory action on arsenite-stimulated glucose uptake. This also implies that the inhibition of p38 MAPK phosphorylation exerted by SB202190 (Figure 1) and by SB203580 (data not shown) does not play a role in inhibition of arsenite-stimulated glucose uptake by these SB's. Furthermore, the noteworthy conclusion can be drawn that both SB's cannot be used to discern the effects of p38 MAPK on glucose uptake. With respect to SB203580, there are recent reports that draw the same conclusion (35, 36), and a putative mechanism of action involves an endofacial interaction with GLUT4. This occupation of the endofacial substrate site of GLUT4 is then supposed to block the return of this substrate site to the extracellular leaflet, causing a drop in V*ₘₐₓ* (33).

Since GLUT4 translocation is entirely responsible for the arsenite-induced increase in glucose uptake, conclusive evidence for the lack of involvement of p38 MAPK in arsenite-stimulated glucose uptake can be deduced from the inability of SB202190 to affect arsenite-induced GLUT4 translocation
*• p42*/*44 ERK and p70 S6K:* Arsenite and insulin resemble each other in that they both moderately stimulated p42/44 ERK as well as p70 S6K by phosphorylation. On the other hand, oligomycin did not enhance phosphorylation of these kinases. The inhibitors PD98059 and rapamycin, under conditions at which they fully reverse agonist-induced ERK or S6K activation, respectively, have no direct inhibitory action on GLUT4 itself. They also did not affect arsenite- or insulin-stimulated glucose uptake. Hence, p42/44 ERK and p70 S6K are not involved in translocation of GLUT4 induced by each of these stimuli.

Taken altogether, our study shows that despite the multiple (pleiotropic) effects of arsenite on selected signaling pathways, these signaling actions are not involved in arsenite's selective stimulation of glucose uptake via GLUT4 translocation. It is well documented that intracellularly stored GLUT4 is divided over two compartments: the recycling endosomes and a non-endosomal GLUT4-specific storage compartment (8, 37, 38). This storage compartment harbours a relatively large pool of GLUT4, and is dedicated to mobilize GLUT4 specifically in response to insulin, and not, for instance, in response to contractions (Figure 8). As reviewed previously (8), it is unlikely that intracellular CD36 is present within the non-endosomal storage compartment. However, colocalization with Rab11 indicates that CD36 is present within the recycling endosomes (39), where it can be mobilized by insulin and by contractions. When insulin is added to cardiac myocytes, both the storage compartment and the recycling endosomes are activated to mobilize GLUT4 transporters through budding of small membrane vesicles. The GLUT4-containing vesicles mobilized from the non-endosomal storage compartment are not expected to migrate directly to the cell surface, but first fuse with the recycling endosomes (38, 40, 41; see Figure 8). Simultaneously, transport vesicles will be assembled from the endosomal membranes for further transport of GLUT4 to the sarcolemma. As recently proposed (8), these endosomally-derived vesicles would not only contain GLUT4, but also endosomally stored CD36. The specific action of arsenite on glucose uptake suggests that the intracellular target of arsenite must reside within the storage compartment rather than in the recycling endosomes (Figure 8). A hypothetical thiol-modification of this target by arsenite would then result in excision of GLUT4 containing vesicles from the storage compartment. In contrast to insulin stimulation, the recycling endosomes are not activated by arsenite to mobilize transport vesicles, forcing the storage-compartment-derived vesicles to bypass the endosomes, and migrate directly to the sarcolemma. Although this proposed mechanism of arsenite action is speculative, it effectively explains arsenite's selective effect on glucose uptake. It also explains the fact that arsenite does not behave as a contraction-mimetic agent such as oligomycin, and finally it explains that arsenite can behave as an insulin-mimetic agent with respect to glucose uptake, independent of the PI3K/Akt signaling system (Figure 8).

The invention therefore also relates to a method as described above wherein a stimulus is provided that consists of the provision of a thiol-modifying agent, such as for example arsenite. Other thiol-modifying agents (i.e., reagents that react with vicinal SH-groups), such as phenylarsine oxide (Fischer Y, Rose H, Thomas J, Deuticke B, Kammermeier H. Phenylarsine oxide and hydrogen peroxide stimulate glucose transport via different pathways in isolated cardiac myocytes. Biochim Biophys Acta 1153: 97-104, 1993), have similar effects on GLUT4 translocation/ glucose uptake and CD36 translocation/ fatty acid uptake by cardiac myocytes. Such compounds will induce only GLUT4 translocation/ glucose uptake and will not affect CD36 translocation/ fatty acid uptake. Such compounds may therefore be used as additional selective glucose-uptake stimulating compounds in the methods according to the invention.

Whereas PI3K has been firmly implicated in stimulation of LCFA uptake by insulin (31), virtually no information is available about the role of MAPK's and S6K's in this insulin action. In the present study, arsenite does not increase LCFA uptake, despite activation of p38 MAPK, p42/44 ERK and p70 S6K. Vice versa, blockade of arsenite signaling by the SB's, PD98059 or rapamycin had no effect on LCFA uptake by cardiac myocytes either under basal conditions, or in the presence of insulin or arsenite. In contrast, wortmannin selectively blocks insulin-stimulated LCFA uptake, confirming earlier observations on the involvement of PI3K in insulin-induced LCFA uptake (24, 25). Hence, these observations firmly exclude a role of MAPK's and S6K's in insulin-induced induction of CD36 translocation and stimulation of LCFA uptake in the heart.

The most important finding in this study is the arsenite-induced selective stimulation of glucose uptake into cardiac myocytes. We found that this selective stimulation of glucose uptake involves a thiol-modification of an arsenite-sensitive protein. The insulin-mimetic action of arsenite on glucose uptake pinpoints this protein within the insulin-responsive storage compartment, harbouring the majority of intracellular GLUT4. The important implication of the intrinsic ability of the heart to selectively upregulate glucose uptake is that this would be favourable under conditions in which cardiac glucose uptake is repressed. Notably in the insulin resistant and diabetic heart there is a marked shift from glucose utilization to LCFA utilization (3) accompanied with a permanent relocation of CD36 to the sarcolemma (42). In the diabetic heart there is also a decreased expression and/or activation of protein kinases (IRS-1, P13K and Akt/PKB) involved in insulin signaling (43, 44). The inability of GLUT4 to be recruited by insulin has been recognized to be responsible for the reduction in cardiac glucose uptake (45). The intracellular target of arsenite involved in stimulation of cardiac glucose uptake presents an ideal target to restore glucose uptake in the diabetic heart. Namely, a direct activation of this arsenite-sensitive target bypasses the defective insulin signaling cascade for recruitment of GLUT4 to the sarcolemma.

The skilled person will appreciate that the mechanism as described herein will apply for th ecardiac muscle as well as the skeletal muscle. The Invention therefore relates to a method as described above that is performed on the human or animal body to restore glucose uptake in the diabetic heart and diabetic skeletal muscle.

Interestingly, environmental exposure to arsenic from drinking water is associated with increased incidence of type-2 diabetes (46). An underlying mechanism for this arsenite-induced type-2 diabetes in humans could include β-cell dysfunction, which is commonly observed upon long-term arsenite exposure, and which is regarded an primary risk factor for this disease (14). Nevertheless, identification of the intracellular target of arsenite involved in selective stimulation of glucose uptake could provide novel information about regulation of recruitment of GLUT4 from its non-endosomal storage compartment. Moreover, it could be the basis of a novel antidiabetic strategy.

Also comprehended by the present invention are antibody substances (e.g., monoclonal and polyclonal antibodies, single chain antibodies, chimeric antibodies, CDR-grafted antibodies and the like) or other binding proteins which are specifically reactive with the ITA as identified in this invention. Antibody substances can be developed using isolated natural or recombinant ITA (including peptides) or cells expressing such products on their surfaces. The antibody substances are useful, in turn, in complexes for immunization to generate anti-idiotypic antibodies as well as for purifying polypeptides of the invention and for identifying cells producing the polypeptides on their surfaces. Assays for the detection and quantification of ITA on cell surfaces and in fluids such as cell culture medium may involve a single antibody substance or multiple antibody substances in a "sandwich" assay format. The antibody substances as well as agonists or antagonists of ITA (e.g., small molecules or peptides) are also manifestly useful in modulating (i.e., blocking, inhibiting or stimulating) ligand/receptor binding reactions of ITA.

### References

1. Stanley WC, Recchia FA, Lopaschuk GD 2005 Myocardial substrate metabolism in the normal and failing heart. Physiol Rev 85:1093-1129
2. Kagaya Y, Kanno Y, Takeyama D, Ishide N, Maruyama Y, Takahashi T, Ido T, Takishima T 1990 Effects of long-term pressure overload on regional myocardial glucose and free fatty acid uptake in rats. Circulation 81:1353-1361
3. Carley AN, Severson DL 2005 Fatty acid metabolism is enhanced in type 2 diabetic hearts. Biochim Biophys Acta 1734:112-126
4. Sharma S, Adrogue JV, Golfman L, Uray I, Lemm J, Youker K, Noon GP, Frazier OH, Taegtmeyer H 2004 Intramyocardial lipid accumulation in the failing human heart resembles the lipotoxic rat heart. FASEB J 18:1692-1700
5. Abumrad NA, el-Maghrabi MR, Amri EZ, Lopez E, Grimaldi PA 1993 Cloning of a rat adipocyte membrane protein implicated in binding or transport of long-chain fatty acids that is induced during preadipocyte differentiation. Homology with human CD36. J Biol Chem 268:17665-17668
6. Stremmel W, Lotz G, Strohmeyer G, Berk PD 1985 Identification, isolation, and partial characterization of a fatty acid binding protein from rat jejunal microvillous membranes. J Clin Invest 75:1068-1076
7. Luiken JJFP, Turcotte LP, Bonen A 1999 Protein-mediated palmitate uptake and expression of fatty acid transport proteins in heart giant vesicles. J Lipid Res 40:1007-1016
8. Luiken JJFP, Coort SLM, Koonen DPY, van der Horst DJ, Bonen A, Zorzano A, Glatz JFC 2004 Regulation of cardiac long-chain fatty acid and glucose uptake by translocation of substrate transporters. Pflugers Arch 448:1-15
9. Luiken JJFP, Coort SLM, Willems J, Coumans WA, Bonen A, Glatz JFC 2004 Dipyridamole alters cardiac substrate preference by inducing translocation of FAT/CD36, but not that of GLUT4. Mol Pharmacol 65:639-645
10. McDowell HE, Walker T, Hajduch E, Christie G, Batty IH, Downes CP, Hundal HS 1997 Inositol phospholipid 3-kinase is activated by cellular stress but is not required for the stress-induced activation of glucose transport in L6 rat skeletal muscle cells. Eur J Biochem 247:306-313
11. Bazuine M, Ouwens DM, Gomes de Mesquita DS, Maassen JA 2003 Arsenite stimulated glucose transport in 3T3-L1 adipocytes involves both Glut4 translocation and p38 MAPK activity. Eur J Biochem 270:3891-3903
12. Liu Q, Hofmann PA 2004 Protein phosphatase 2A-mediated cross-talk between p38 MAPK and ERK in apoptosis of cardiac myocytes. Am J Physiol Heart Circ Physiol 286:H2204-2212
13. Wang X, Proud CG 1997 p70 S6 kinase is activated by sodium arsenite in adult rat cardiomyocytes: roles for phosphatidylinositol 3-kinase and p38 MAP kinase. Biochem Biophys Res Commun 238:207-212
14. Tseng CH 2004 The potential biological mechanisms of arsenic-induced diabetes mellitus. Toxicol Appl Pharmacol 197:67-83
15. Namgung U, Xia Z 2000 Arsenite-induced apoptosis in cortical neurons is mediated by c-Jun N-terminal protein kinase 3 and p38 mitogen-activated protein kinase. J Neurosci 20:6442-6451
16. Sweeney G, Somwar R, Ramlal T, Volchuk A, Ueyama A, Klip A 1999 An inhibitor of p38 mitogen-activated protein kinase prevents insulin-stimulated glucose transport but not glucose transporter translocation in 3T3-L1 adipocytes and L6 myotubes. J Biol Chem 274:10071-10078
17. Li J, Miller EJ, Ninomiya-Tsuji J, Russell RR 3rd, Young LH 2005 AMP-activated protein kinase activates p38 mitogen-activated protein kinase by increasing recruitment of p38 MAPK to TAB1 in the ischemic heart. Circ Res 97:872-879
18. Pelletier A, Joly E, Prentki M, Coderre L 2005 Adenosine 5'-monophosphate-activated protein kinase and p38 mitogen-activated protein kinase participate in the stimulation of glucose uptake by dinitrophenol in adult cardiomyocytes. Endocrinology 146:2285-2294
19. Turcotte LP, Raney MA, Todd MK 2005 ERK1/2 inhibition prevents contraction-induced increase in plasma membrane FAT/CD36 content and FA uptake in rodent muscle. Acta Physiol Scand 184:131-139
20. Chen Y, Rajashree R, Liu Q, Hofmann P 2003 Acute p38 MAPK activation decreases force development in ventricular myocytes. Am J Physiol Heart Circ Physiol 285:H2578-H2586
21. Mohammadi K, Liu L, Tian J, Kometiani P, Xie Z, Askari A 2003 Positive inotropic effect of ouabain on isolated heart is accompanied by activation of signal pathways that link Na+/K+-ATPase to ERK1/2. J Cardiovasc Pharmacol 41:609-614
22. Luiken JJFP, van Nieuwenhoven FA, America G, van der Vusse GJ, Glatz JFC 1997 Uptake and metabolism of palmitate by isolated cardiac myocytes from adult rats: involvement of sarcolemmal proteins. J Lipid Res 38:745-758
23. Fischer Y, Thomas J, Rosen P, Kammermeier H 1995 Action of metformin on glucose transport and glucose transporter GLUT1 and GLUT4 in heart muscle cells from healthy and diabetic rats. Endocrinology 136:412-420
24. Luiken JJFP, Koonen DPY, Willems J, Zorzano A, Becker C, Fischer Y, Tandon NN, Van Der Vusse GJ, Bonen A, Glatz JFC 2002 Insulin stimulates long-chain fatty acid utilization by rat cardiac myocytes through cellular redistribution of FAT/CD36. Diabetes 51:3113-3119
25. Chabowski A, Coort SLM, Calles-Escandon J, Tandon NN, Glatz JFC, Luiken JJFP, Bonen A 2004 Insulin stimulates fatty acid transport by regulating expression of FAT/CD36 but not FABPpm. Am J Physiol Endocrinol Metab 287:E781-E789
26. Luiken JJFP, Coort SLM, Willems J, Coumans WA, Bonen A, van der Vusse GJ, Glatz JFC 2003 Contraction-induced fatty acid translocase/CD36 translocation in rat cardiac myocytes is mediated through AMP-activated protein kinase signaling. Diabetes 52:1627-1634
27. Somwar R, Koterski S, Sweeney G, Sciotti R, Djuric S, Berg C, Trevillyan J, Scherer PE, Rondinone CM, Klip A 2002 A dominant-negative p38 MAPK mutant and novel selective inhibitors of p38 MAPK reduce insulin-stimulated glucose uptake in 3T3-L1 adipocytes without affecting GLUT4 translocation. J Biol Chem 277:50386-50395
28. Koonen DPY, Coumans WA, Arumugam Y, Bonen A, Glatz JFC, Luiken JJFP 2002 Giant membrane vesicles as a model to study cellular substrate uptake dissected from metabolism. Mol Cell Biochem 239:121-130.
29. Chabowski A, Coort SLM, Calles-Escandon J, Tandon NN, Glatz JFC, Luiken JJFP, Bonen A 2005 The subcellular compartmentation of fatty acid transporters is regulated differently by insulin and by AICAR. FEBS Lett 579:2428-2432
30. Rakatzi I, Ramrath S, Ledwig D, Dransfeld O, Bartels T, Seipke G, Eckel J 2003 A novel insulin analog with unique properties: LysB3,GluB29 insulin induces prominent activation of insulin receptor substrate 2, but marginal phosphorylation of insulin receptor substrate 1. Diabetes 52:2227-2238
31. Watson RT, Pessin JE 2001 Subcellular compartmentalization and trafficking of the insulin-responsive glucose transporter, GLUT4. Exp Cell Res 271:75-83
32. Fujishiro M, Gotoh Y, Katagiri H, Sakoda H, Ogihara T, Anai M, Onishi Y, Ono H, Funaki M, Inukai K, Fukushima Y, Kikuchi M, Oka Y, Asano T 2001 MKK6/3 and p38 MAPK pathway activation is not necessary for insulin-induced glucose uptake but regulates glucose transporter expression. J Biol Chem 276:19800-19806.
33. Ribe D, Yang J, Patel S, Koumanov F, Cushman SW, Holman GD 2005 Endofacial competitive inhibition of glucose transporter-4 intrinsic activity by the mitogen-activated protein kinase inhibitor SB203580. Endocrinology 146:1713-1717.
34. lijima Y, Laser M, Shiraishi H, Willey CD, Sundaravadivel B, Xu L, McDermott PJ, Kuppuswamy D 2002 c-Raf/MEK/ERK pathway controls protein kinase C-mediated p70S6K activation in adult cardiac muscle cells. J Biol Chem 277:23065-23075.
35. Bazuine M, Carlotti F, Rabelink MJ, Vellinga J, Hoeben RC, Maassen JA 2005 The p38 mitogen-activated protein kinase inhibitor SB203580 reduces glucose turnover by the glucose transporter-4 of 3T3-L1 adipocytes in the insulin-stimulated state. Endocrinology 146:1818-1824
36. Turban S, Beardmore VA, Carr JM, Sakamoto K, Hajduch E, Arthur JS, Hundal HS 2005 Insulin-stimulated glucose uptake does not require p38 mitogen-activated protein kinase in adipose tissue or skeletal muscle. Diabetes 54:3161-3168
37. Aledo JC, Lavoie L, Volchuk A, Keller SR, Klip A, Hundal HS 1997 Identification and characterization of two distinct intracellular GLUT4 pools in rat skeletal muscle: evidence for an endosomal and an insulin-sensitive GLUT4 compartment. Biochem J 325:727-732
38. Govers R, Coster AC, James DE 2004 Insulin increases cell surface GLUT4 levels by dose dependently discharging GLUT4 into a cell surface recycling pathway. Mol Cell Biol 24:6456-6466
39. Kessler A, Tomas E, lmmler D, Meyer HE, Zorzano A, Eckel J 2000 Rab11 is associated with GLUT4-containing vesicles and redistributes in response to insulin. Diabetologia 43:1518-1527
40. Hashiramoto M, James DE 2000 Characterization of insulin-responsive GLUT4 storage vesicles isolated from 3T3-L1 adipocytes. Mol Cell Biol 20:416-427
41. Hah JS, Ryu JW, Lee W, Kim BS, Lachaal M, Spangler RA, Jung CY 2002 Transient changes in four GLUT4 compartments in rat adipocytes during the transition, insulin-stimulated to basal: implications for the GLUT4 trafficking pathway. Biochemistry 41:14364-14371
42. Coort SLM, Hasselbaink DM, Koonen DPY, Willems J, Coumans WA, Chabowski A, van der Vusse GJ, Bonen A, Glatz JFC, Luiken JJFP 2004 Enhanced sarcolemmal FAT/CD36 content and triacylglycerol storage in cardiac myocytes from obese zucker rats. Diabetes 53:1655-1663
43. Kim YB, Ciaraldi TP, Kong A, Kim D, Chu N, Mohideen P, Mudaliar S, Henry RR, Kahn BB 2002 Troglitazone but not metformin restores insulin-stimulated phosphoinositide 3-kinase activity and increases p110beta protein levels in skeletal muscle of type 2 diabetic subjects. Diabetes 51:443-448
44. Desrois M, Sidell RJ, Gauguier D, King LM, Radda GK, Clarke K 2004 Initial steps of insulin signaling and glucose transport are defective in the type 2 diabetic rat heart. Cardiovasc Res 61:288-296
45. Uphues I, Kolter T, Goud B, Eckel J 1995 Failure of insulin-regulated recruitment of the glucose transporter GLUT4 in cardiac muscle of obese Zucker rats is associated with alterations of small-molecular-mass GTP-binding proteins. Biochem J 311:161-166
46. Tseng CH, Tai TY, Chong CK, Tseng CP, Lai MS, Lin BJ, Chiou HY, Hsueh YM, Hsu KH, Chen CJ 2000 Long-term arsenic exposure and incidence of non-insulin-dependent diabetes mellitus: a cohort study in arseniasis-hyperendemic villages in Taiwan. Environ Health Perspect 108:847-851

### Examples

### Example 1: Materials

[1-¹⁴C]palmitic acid and [³H]deoxyglucose were obtained from Amersham Life Science Ltd., Little Chalfont, U.K. Bovine serum albumin (BSA) (fraction V), collagenase type VII, insulin, oligomycin, sodium arsenite, wortmannin, SB202190, SB203580, PD98059 and rapamycin were purchased from Sigma, Saint Louis, Missouri. Collagenase type II was from Worthington (Freehold, New Jersey). Non-fat dry milk (Marvel) was obtained from Premier Brands, Moreton, UK. Western blot reagents were from Bio-Rad Laboratories (Hercules, CA) and the enhanced chemiluminescence (ECL) kit was from Amersham Pharmacia Biotech (Buckingham, UK). CD36 was detected with a monoclonal antibody (MO25) directed against human CD36, kindly provided by Dr. N. Tandon, Otsuka Pharmaceuticals, Bethesda MD. A rabbit polyclonal against rat hepatic membrane fatty acid binding protein was used to detect FABPpm (gift from Dr. J Calles-Escandon, Section of Endocrinology and Metabolism, Wake Forest University School of Medicine and Baptist Medical Center, Winston-Salem, North Carolina.). Antibodies directed against GLUT4 were obtained from Sanver Tech (Heerhugowaard, the Netherlands). Phosphospecific antibodies against signaling enzymes were from Cell Signaling (City, State). Rabbit anti-mouse immunoglobulin horseradish peroxidase and pork anti-rabbit immunoglobulin horseradish peroxidase were obtained from DAKO (Glostrup, Denmark).

### Example 2: Isolation of cardiac myocytes

Cardiac myocytes were isolated from male Lewis rats (200-250 g) using a Langendorff perfusion system and a Krebs-Henseleit bicarbonate medium supplemented with 11 mM glucose, and equilibrated with a 95% O₂ and 5% CO₂ gas phase (medium A) at 37°C as previously described (22). After isolation, the cells were washed twice with medium A supplemented with 1.0 mM CaCl₂ and 2 % (w/v) BSA (medium B) and then suspended in 15 ml of medium B. The isolated cells were allowed to recover for approximately 2 h at room temperature. At the end of the recovery period, cells were washed and suspended in medium B. Only when >80% of the cells had a rod-shaped appearance and excluded trypan blue, were they used for subsequent tracer uptake studies.

### Example 3: Substrate uptake by cardiac myocytes

Cells (2.0 ml; 5-8 mg wet mass/ml), suspended in medium B without glucose, were preincubated in capped 20-ml incubation vials for 15 min at 37°C under continuous shaking. To study palmitate uptake, 0.5 ml of the [1-¹⁴C]palmitate/BSA complex was added at the start of the incubations so that the final concentration of palmitate amounted to 100 µM with a corresponding palmitate/BSA ratio of 0.3. This palmitate/BSA complex was prepared as previously described (22). To study deoxyglucose uptake, [³H]deoxyglucose was added at the start of the incubations in 0.6 ml medium B without glucose to a final concentration of 100 µM. Cellular uptake of palmitate (3 min incubation) and of deoxyglucose (3 min incubation) were determined upon washing the cells three times for 2 min at 100xg in an ice-cold stop solution containing 0.2 mM phloretin as previously described (22). The washing procedure did not affect cellular integrity as evaluated by microscopical inspection.

Substrate uptake was stimulated by sodium arsenite (<0.3 mM), insulin (10 nM) and oligomycin (30 µM), These stimuli were added to the cell incubations 15 min prior to addition of radiolabeled substrate. A stock solution of oligomycin was prepared in DMSO, which never exceeded a final concentration of 0.5% in the cell suspensions. At this concentration, DMSO did not affect cellular substrate utlization.

### Example 4: Measurement of activation of signaling kinases

Cardiac myocytes (8-12 mg wet mass/ml) were incubated in medium B in the absence and presence of additions for 15 min. At the end of the incubation, cells were pulse-centrifuged in an Eppendorf microfuge, and then immediately dissolved in sample buffer containing 15 mM Tris-HCl (pH 6.8), 0.5 mM EDTA, 5 mM dithiothreitol and 2% (w/v) SDS, and used for SDS-polyacrylamide gel electrophoresis. Activation of p38 MAPK, p42/44 ERK, p70 S6K, Akt/PKB, AMPK and acetyl-CoA carboxylase (ACC) was measured with phosphospecific monoclonal antibodies from Cell Signaling Technology. For this purpose, antibodies against phospho-p38 MAPK (Thr180/Tyr182), phospho-p42/44 ERK (Thr202/Tyr204), phospho-p70 S6K (Thr389), phospho-Akt (Ser473), phospho-AMPKα (Thr172) and phospho-ACC were used according to the manufacturer's instructions.

In order to link activation of these kinases to their effects on metabolism a variety of signaling inhibitors were used; i.e., the p38 MAPK inhibitors SB203580 and SB202190, the p42/44 ERK inhibitor PD98059, the p70 S6K inhibitor rapamycin and the PI3K inhibitor wortmannin (200 nM). But prior to their use in substrate uptake studies, these inhibitors were tested whether they exerted their desired inhibitory effect on signaling in cardiac myocytes and whether this inhibitory effect was specific for one protein kinase.

All inhibitors were added at the lowest concentration at which they exerted their maximal effect. None of these agents, alone or in combination with pharmacological stimuli were found to affect the percentage of cells that (i) were rod-shaped and (ii) excluded trypan blue, as parameters of cellular integrity.

At 10 µM, SB202190 largely inhibited arsenite-stimulated p38 MAPK phosphorylation, but did not affect phenylephrine-induced p42/44 ERK phosphorylation, nor arsenite-induced p70 S6K phosphorylation, nor insulin-induced Akt/PKB phosphorylation, nor oligomycin-induced ACC phosphorylation (Figure 1). SB203580, at the same concentration used as SB202190, was similarly potent in blocking p38 MAPK phosphorylation (data not shown). PD98059 (10 µM) completely inhibited phenylephrine-induced p42/44 ERK phosphorylation, but did not inhibit arsenite-induced p38 MAPK- or p70 S6K phosphorylation, nor insulin-induced Akt/PKB phosphorylation, nor oligomycin-induced ACC phosphorylation (Figure 1). Rapamycin (20 nM) solely inhibited arsenite-induced S6K phosphorylation without altering phosphorylation of the other kinases upon stimulation (Figure 1). Finally, wortmannin (200 nM) completely blocked insulin-stimulated Akt/PKB- and arsenite-induced p70 S6K phosphorylation, and left phenylephrine-induced p42/44 ERK- and oligomycin-induced ACC phosphorylation unaltered (Figure 1). Because Akt/PKB and p70 S6K are recognized as protein kinases downstream of PI3K, this inhibitory action of wortmannin on both kinases is not surprising. However, when considered that p70 S6K can also be activated in a P13K-independent manner, the observation that arsenite-stimulated S6K phosphorylation is wortmannin sensitive is a novel side-observation. Taken together, at the concentrations used all inhibitors exerted their claimed specific effects on signaling in cardiac myocyte incubations.

### Example 5: Subcellular fractionation of cardiac myocytes

Cardiac myocytes (2,25 ml; 20-25 mg wet mass/ml) were incubated for 15 min in medium B in the absence and presence of additions. At the end of the incubation the total volume of cell incubations was quickly transferred to a tightly fitting 5-ml Potter-Elvejhem glass homogenizer on ice containing 1 ml of cold H₂O, after which NaN₃ was added to a final concentration of 5 mM in order to stop ATP-dependent vesicular trafficking events. Immediately hereafter cell suspensions were homogenized with 10 strokes. Subsequently, fractionation was carried out as described previously (23, 24). For determination of the GLUT4, CD36 and FABPpm content in the plasma membrane (PM) and in low-density microsomes (LDM), aliquots of the membrane fractions were separated with SDS-polyacrylamide gel electrophoresis and Western blotting as previously described (7, 24, 25).

The purity of the fractions obtained by this fractionation procedure was previously checked (23). Specifically, the PM fraction is 13.5-fold enriched with ouabain-sensitive p-nitrophenyl-phosphatase, whereas the specific activity of the sarcoplasmatic EGTA-sensitive Ca²⁺-ATPase was 3.6-fold decreased. In addition, no activity of p-nitrophenyl-phosphatase or of Ca²⁺-ATPase could be detected in the LDM fraction, indicating that this fraction was devoid of plasma membrane and of sarcoplasmic reticulum. Furthermore, caveolin-3 was found to be 2.9-fold more abundant in the PM fraction than in the LDM fraction (data not shown).

### Example 6: Isolation of giant vesicles

Giant vesicles were prepared from rat heart, as described previously (7). In general, tissues were cut into thin layers (-1-3 mm thick) and incubated in vesicle preparation medium (140 mM KCI/10 mM MOPS; pH 7.4) supplemented with aprotinin (10 mg/ml), 0.8 mM CaCl₂ and collagenase type II (0.3%, w/v) for 1 h at 34°C in a shaking waterbath. Following incubation, tissues were washed with KCI/MOPS and 10 mM EDTA. The supernatant was collected and Percoll® (final concentration of 16% (v/v)) and aprotinin (1.0 mg/ml) were added. The resulting suspension was placed at the bottom of a density gradient consisting of a 3 ml middle layer of 4% Nycodenz (w/v) and a 1 ml KCI/MOPS upper layer and centrifugated at 60 g for 45 min at room temperature. Subsequently, the vesicles were harvested from the interface of the upper and middle layer, diluted in KCI/MOPS, recentrifugated at 13,000 g for 4 min, and finally resuspended in KCI/MOPS. For each assay, a protein concentration of 0.25-0.60 mg/ml was used. Vesicles were immediately used for glucose- and LCFA transport experiments.

### Example 7: Substrate uptake by giant vesicles

Palmitate uptake was measured by addition of unlabeled and radiolabeled 0.3 µCi [9,10-³H]palmitate and 0.06 µCi [¹⁴C]mannitol in a 0.1 % BSA KCL/MOPS solution to 40 µl of vesicles (~80 µg protein). The reaction was carried out at room temperature for precisely 15 s. Palmitate uptake was terminated by addition of 1.4 ml of ice-cold KCL/MOPS-2.5 mM HgCl and 0.1 % BSA. The sample was quickly recentrifuged at maximal speed in a microcentrifuge for 2 min. The supernatant was discarded, and radioactivity was measured in the tip of the tube. Non-specific uptake was measured by adding the stop solution prior to the addition of the radiolabeled palmitate solution.

Glucose uptake studies were carried out by addition of 40 µl 0.1% BSA in KCI/MOPS containing 0.3 µCi [³H]D-glucose (200 µM) and 0.06 µCi [¹⁴C]mannitol. Incubations were carried out at room temperature for 1 min. The incubation was ended and radioactivity was determined as described above.

Arsenite and signaling inhibitors were added to giant vesicles 20 min prior to determination of palmitate or glucose uptake at the same concentrations as applied for cardiac myocyte studies (see above).

### Example 8: Other procedures

Cellular wet mass was obtained from cell samples taken during the incubation period and determined after centrifugation for 2-3 s at maximal speed in a microcentrifuge and subsequent removal of the supernatant. Protein was quantified with the Bicinchichonic acid protein assay (Pierce, Rockford, IL, USA) according to manufacturer's instructions.

### Example 9: Statistics

All data are reported as mean ± SEM. Statistical difference between groups was tested with a Student's *t*-test. However, when the data failed to meet a test of normal distribution, we necessarily employed the Mann-Whitney U test. P values equal to or less than 0.05 were considered significant.

### Example 10: Effects of arsenite on glucose and LCFA uptake into cardiac myocytes

Arsenite has been previously shown to stimulate glucose uptake into myocytes (10) and adipocytes (11), but it has never been used to study cellular LCFA uptake. Using cardiac myocytes, arsenite stimulated deoxyglucose uptake in a dose-dependent manner up to 5.1-fold at 100 µM, without a further increase at 300 µM (Figure 2). In contrast, arsenite did not increase LCFA uptake even at concentrations up to 300 µM (Figure 2). Therefore, we chose a concentration of 100 µM to study arsenite's effects on cardiac glucose and fatty acid utilization and signaling.

We have previously demonstrated that contractile activity and insulin are two major physiological stimuli enhancing uptake of both glucose and LCFA (24, 26). Oligomycin, an inhibitor of the mitochondrial proton pump, was able to mimick the contraction-induced increase in substrate utilization (26). In the present study, glucose uptake was increased upon addition of insulin (10 nM) and of oligomycin (5 µM) by 7.2-fold and 2.9-fold, respectively (Figs. 2 and 3). More importantly, arsenite retained its stimulatory action on glucose uptake when added in combination with oligomycin, However, arsenite was ineffective on glucose uptake in insulin-stimulated cardiac myocytes (Figure 3). Hence, the effect of arsenite on glucose uptake was additive to that of oligomycin, but non-additive to that of insulin. LCFA uptake was increased in the presence of insulin and oligomycin by 1.5-fold and 2.0-fold respectively. In combination with insulin or oligomycin, arsenite did not significantly alter LCFA uptake (Figure 3).

### Example 11: Effects of blocking of signaling pathways on the uptake of glucose and LCFA

In order to dissect the signaling mechanisms involved in the selective stimulatory effect of arsenite on glucose uptake, various signaling inhibitors were used at their optimal concentrations (see Materials and Methods); i.e., the p38 MAPK inhibitors SB203580 (10 µM) and SB202190 (10 µM), the p42/44 ERK inhibitor PD98059 (10 µM), the p70 S6K inhibitor rapamycin (100 nM) and the P13K inhibitor wortmannin (200 nM). PD98059 and rapamycin had no effect on basal glucose uptake. Similarly, neither PD98059 nor rapamycin affected glucose uptake in the presence of any other stimuli (Figure 4). In contrast, basal glucose uptake was however markedly inhibited by SB202190 (-45%) (Figure 4). Similarly, SB202190 also inhibited glucose uptake to a comparable extent in the presence of either insulin (-33%) or arsenite (-42%), The inhibitor SB203580 also inhibited basal glucose uptake (-56%), and glucose uptake in the presence of insulin (-46%) or arsenite (-57%), effects that were similar to those of SB202190. It was only when the PI3-kinase inhibitor wortmannin was used that the differing effects of insulin and arsenite on glucose uptake became evident. While wortmannin did not inhibit basal glucose transport, insulin-stimulated glucose uptake was largely blocked by wortmannin. Remarkably, wortmannin completely failed to inhibit the arsenite-stimulated glucose uptake.

With respect to LCFA uptake, neither SB202190, nor PD98059 or rapamycin were able to influence the uptake of this substrate into cardiac myocytes, whether in the absence or in the presence of insulin or arsenite (Figure 4). However, wortmannin, while having no effect on basal LCFA uptake, completely blocked insulin-induced LCFA uptake, in agreement with our earlier observations (24, 25).

### Example 12: Effects of arsenite on intrinsic activity of transporters in giant vesicles:

It is generally accepted the increase in glucose uptake into myocytes and adipocytes in the presence of physiological stimuli can largely be explained by a translocation of GLUT1 and/or GLUT4 from intracellular stores to the plasma membrane. An additional mechanism by which glucose uptake can be increased, includes an increase in the intrinsic activity of GLUT1 and/or GLUT4, as reported by Klip and coworkers (16, 27). In order to explore the possibility that arsenite stimulates glucose uptake through activation of transporters already located at the plasma membrane, giant vesicles were used for measurement of glucose uptake. These vesicles are formed by budding of the cardiomyocytic sarcolemma during incubation of cardiac tissue in KCI-containing buffers. During this budding process, cytoplasmic areas become sequestered within these giant vesicles. However, intracellular organelles such as the endosomal recycling compartment are absent (28), so that translocation of transporters cannot occur. Hence, during incubation of giant vesicles with selected stimuli, the number of transporters present at the vesicular membrane remains fixed, and therefore, alterations in transport rates can only be due to changes in the activities of transporters. Previously, we found that a pharmacological stimulus (i.e., isobutyl-methylxanthine) is able to increase substrate transporter activity in giant vesicles (28). In the present study, arsenite did not influence either glucose uptake or LCFA uptake, thereby indicating that arsenite does not interact directly with GLUT4 or any LCFA transporters (Figure 5).

When dissecting the substrate uptake-related effects of the signaling inhibitors in giant vesicles, it was observed that none of these inhibitors affected LCFA uptake. Similarly, PD98059, rapamycin and wortmannin also did not alter glucose uptake into giant vesicles. However, SB202190 (-27%) inhibited glucose uptake into giant vesicles (Figure 5) to a similar extent as was observed in cardiac myocytes (-33%) (Figure 4). Also SB203580 inhibited glucose uptake into giant vesicles (-46%) to the same extent as in cardiac myocytes (-56%). Hence, these latter compounds directly inhibit glucose transporter activity at the plasma membrane.

### Example 13: Effects of arsenite on the translocation of GLUT4 and LCFA transporters

Because arsenite does not influence glucose transporter activity at the sarcolemma, an alternative explanation for the stimulation of glucose uptake by arsenite involves the translocation of GLUT4 to the sarcolemma. In order to investigate this notion, cardiac myocytes were incubated with arsenite for 20 min prior to subcellular fractionation. As a positive control for the fractionation procedure, parallel myocyte incubations were performed with insulin for 20 min. Indeed insulin decreased the contents of GLUT4 and CD36 in the LDM fraction by 41 % and by 42%, respectively, and simultaneously increased the contents of both transporters in the PM fraction by 1.9-fold and 1.5-fold, respectively (Figure 6). In contrast, insulin did not affect the distribution of FABPpm between the LDM and the PM fraction. Because the insulin-induced translocation of GLUT4 is among the best-established intracellular trafficking events, our fractionation procedure is suitable for measuring stimulus-induced translocation processes. The insulin-induced translocation of CD36 and the inability of insulin to influence the subcellular recycling of FABPpm are in agreement with our previous findings (24, 29). In agreement with insulin's effects on GLUT4 distribution, arsenite decreased the content of GLUT4 in the LDM fraction by 44%, and concomitantly increased its content in the PM fraction by 1.7-fold (Figure 6). SB202190 did not affect this arsenite-induced GLUT4 translocation (Figure 6, insert). With respect to LCFA transporter distribution, arsenite did not significantly influence the contents of CD36 and FABPpm in the LDM- and PM fractions. In addition, the inclusion of SB202190 did not influence the subcellular distribution of both LCFA transporters (Figure 6, inserts). When compared to insulin's effects on transporter recycling, arsenite is almost as potent in inducing the translocation of GLUT4, but in contrast, arsenite completely fails to have any stimulatory effect on inducing the translocation of CD36.

### Example 14: Effects of arsenite on cardiac signaling

Arsenite is considered a useful tool to investigate whether there is a relation between MAPK signaling and substrate utilization (11). The ability of arsenite to stimulate MAPK and other key signaling kinases was compared with that of insulin and oligomycin, two stimuli with profound effects on cardiac substrate uptake. Our focus was (i) on the phosphorylation of MAPKs and of S6K, known to be stimulated by arsenite (13), (ii) on the phosphorylation of Akt/PKB, a hallmark for insulin signaling, and (iii) on the phosphorylation of AMPK and ACC, key protein kinases in contraction signaling. As expected, incubation of cardiac myocytes with insulin during 20 min resulted in a large (>10-fold) increase in phosphorylation of Akt/PKB and a marked (2.8 ± 0.4-fold) increase in p70 S6K phosphorylation (Figure 7). In addition, there was also a modest (1.8 ± 0.3-fold) increase in p42/44 ERK phosphorylation, which has also been previously reported in cardiac myocytes (30). Insulin, as expected, had no effect on the phosphorylation of AMPK and ACC. In contrast, incubation of cardiac myocytes with oligomycin for 20 min strongly induced the phosphorylation of AMPK and ACC (both >10-fold). Concomitantly, there was also a markedly enhanced phosphorylation of p38 MAPK (5.7 ± 1.5-fold), but oligomycin did not increase the phosphorylation state of p42/44 ERK and Akt/PKB (Figure 7).

The incubation of cardiac myocytes with arsenite for 20 min, led to a potent induction of p38 MAPK phosphorylation (7.8 ± 1.8 fold; Figure 7), a well known effect of arsenite. However, p70 S6K phosphorylation was also appreciable (3.3 ± 0.7-fold). This effect had also been observed previously in cardiac myocytes (13). Finally, arsenite also induced mildly the phosphorylation of p42/44 ERK (2.3 ± 0.5-fold), whereas the phosphorylation of AMPK and ACC remained unaltered.

The following procedures are constructively reduced to practice and therefore described in the present tense, as opposed to the previous examples which were carried out in the laboratory and which are therefore set forth in the past tense.

### Example 15: Identification of the intracellular target of arsenite.

The intracellular target of arsenite may be identified using techniques known in the art and known to the skilled person.

In brief, the following procedure may lead to a successful identification of this intracellular target. Isolated cardiac myocytes may be incubated with or without arsenite or any other suitable thiol-modifying agent. The cells from each of these incubations may then be fractionated applying standard biochemical methods such as differential centrifugation, for instance into a membrane fraction, isolated intracellular organelles (mitochondria, peroxisomes, etc) and soluble proteins. Alternatively, the non-endosomal GLUT4-specific storage compartment may be immunoprecipitated by using antibodies directed against insulin-responsive aminopeptidase (IRAP), which is a specific protein for this storage compartment. In yet another alternative, the protein-containing subcellular fractions described above, may be subjected to affinity chromatography on an arsenite-Sepharose 4B column. Arsenite-binding proteins will be retained on the column; subsequent washing of the column with an arsenite-containing buffer will elute these proteins.

Each of the fractions thus obtained may then be subjected to two-dimensional (2D-) gel-electrophoresis, followed by visualization of the protein spots by staining with a fluorescent dye with high dynamic range. As an alternative approach, radiolabeled forms of the thiol-modifying agents may allow the identification of the spots in 2-D gels using autoradiography detection. Protein spot patterns from cells incubated with and without a thiol-modifying agent may also be compared by imaging software. Spots from differentially expressed proteins under the influence of the agent may thus be readily identified. One may even choose to use multiple thiol-modifying agents at the same time and select those spots that occur in fractions of all thiol-modifying agents. The protein(s) present in the spots thus identified may be eluted from the gel and analyzed by established mass-spectrometry (MALDI-TOF MS; LC-MS/MS) to obtain peptide/aminoacid information. Relevant sequence data may be obtained from public data banks, full length proteins may then be obtained by a routine cloning.

The validity of the target of arsenite thus identifed can be verified by downregulating the amount of protein in cardiac myocytes (or a suitable cell line) using a standard siRNA approach and testing whether in these cells, with this downregulated protein, the arsenite-induced selective increase in GLUT4 translocation/ glucose uptake is blunted. In this way ITA may be easily identified using only routine skills and equipment.

### Example 16; isolation and purification of ITA

The polypeptide obtained in example 15 may be produced by first cloning the corresponding polynucleotides into a mammalian expression vector using established protocols that are familiar to those in skill of the art. In brief, polynucleotides of the invention are amplified and isolated via agarose gel electrophoresis and gel excision. The polynucleotides are subsequently cloned into appropriate vectors to facilitate in-frame cloning. Proteins may for instance be expressed with a polyhistidine metal-binding tag. The vectors with polynucleotides of the invention inserted can then be transfected into a mammalian cell line like COS-7 for high-level expression of the corresponding polypeptides.

The polypeptides useful in the invention that are produced by the transfected mammalian cell line can then be purified according to established protocols that are familiar to those of skill in the art, for example using the Ni-NTA Magnetic Agarose Beads protein purification protocol, Qiagen, Valencia, Calif. In brief, assays utilizing Ni-NTA Magnetic Beads involve capture of the His-tagged protein-from a cell lysate or a purified-protein solution-followed by washing, binding of interaction partners, further washing, and finally elution of the interacting partner from the still immobilized His-tagged protein or elution of the interacting-partner-His-tagged-protein complex. Between each step, the beads are collected by attracting them to the side of the vessel, after placing near a magnet for 30-60 seconds. Purification procedures may even use crude cell extracts for binding of His-tagged protein.

The resulting purified polypeptides of the invention are then quantified, lyophilized and stored at 4 degrees Celcius or below, and may be used for the generation of specific antibodies or for identifying further agonists or antagonists that are useful in the methods according to the invention.

Alternatively, the ITA may be expressed on the surface of a cell preferably an eukaryotic cell and thus used in a high throughput screening assay.

### Example 17 identification of compounds that interact with ITA

ITA as identified and isolated above in example 16 may conveniently be used to find agonists or antagonists that may be used in the method according to the invention. The skilled person will be aware of techniques that can be used to reach that target. In brief: a high screening throughput assay may be obtained by immobilizing purified ITA on a solid support, either as a purified protein or as a protein expressed at the surface of a cell. A library of compounds can then be tested for binding or induction of conformational change. Suitable systems are readily available in the art, preferably however, a two-hybrid system is used (Fields S, Song O-K (1989) A novel genetic system to detect protein-protein interactions. Nature 340: 245-246).

## Claims

1. Method for specifically increasing the glucose uptake in a muscle cell wherein a stimulus is provided to said muscle cell upon which stimulus the major cardiac glucose transporter GLUT4 is translocated from the GLUT4 storage compartment directly to the sarcolemma, thereby by-passing the insulin-responsive recycling endosomes, wherein said stimulus does not alter the uptake of long-chain fatty acids in said muscle cell.

2. Method according to claim 1 wherein said stimulus targets the intracellular target of arsenite residing in or closely asscoiated with the GLUT4 storage compartment.

3. Method according to claims 1 or 2 wherein said stimulus consists of the provision of a thiol-modifying agent.

4. Method according to claims 1 or 2 wherein said stimulus is the provision of arsenite.

5. Method according to claims 1 - 4 performed on the human or animal body to restore glucose uptake in the diabetic heart and diabetic skeletal muscle.
